(19) Europäisches Patentamt
European Patent Office
Office européen des brevets

(11) Publication number: **0 122 707**
**B1**

(12) # EUROPEAN PATENT SPECIFICATION

(45) Date of publication of patent specification: **16.09.87**

(21) Application number: **84301463.0**

(22) Date of filing: **06.03.84**

(51) Int. Cl.[4]: **C 07 H 19/04,** A 61 K 31/70, C 07 D 307/32, C 07 H 11/00 // C07D317/30

(54) **Improvements in or relating to novel difluoro antiviral agents.**

(30) Priority: **10.03.83 US 473883**

(43) Date of publication of application:
**24.10.84 Bulletin 84/43**

(45) Publication of the grant of the patent:
**16.09.87 Bulletin 87/38**

(84) Designated Contracting States:
**AT BE CH DE FR GB IT LI LU NL SE**

(58) References cited:
**EP-A-0 010 205**

**CHEMICAL ABSTRACTS, vol. 75, no. 15, 11th October 1971, page 335, no. 98773a, Columbus, Ohio, USA; J. ADAMSON et al.: "2-Deoxy-2,2-difluoro-D-arabino-hexose (2,2-difluoro-glucose)"**

**The file contains technical information submitted after the application was filed and not included in this specification**

(73) Proprietor: **ELI LILLY AND COMPANY**
**307, East McCarty Street**
**Indianapolis Indiana 46285 (US)**

(72) Inventor: **Hertel, Larry Wayne**
**912, Whidbey Court**
**Indianapolis Indiana 46229 (US)**

(74) Representative: **Crowther, Terence Roger et al**
**Erl Wood Manor**
**Windlesham Surrey GU20 6PH (GB)**

**EP 0 122 707 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention provides a new difluoro carbohydrate and new antiviral nucleosides prepared by coupling the new carbohydrate with appropriate bases.

Researchers have long known that antiviral drugs can be found among the general family of nucleosides. For example, 5-(2-bromovinyl)-2′-deoxyuridine is known to be effective against herpes virus. DeClercq, *et al., Proc. Natl. Acad. Sci. USA 76*, 2947—51 (1979). Watanabe, *et al.*, have described a number of nucleosides formed by coupling 2-fluoro-2-deoxyarabinofuranose with cytosine and thymine bases; of these compounds, 5-iodocytosine was the most preferred base. *J. Med. Chem. 22*, 21—24 (1979), and U.S. Patent 4,211,773.

A compound described as an acyclic nucleoside, 9-(2-hydroxyethoxymethyl)guanine, is a potent antiviral agent useful against herpes viruses, and is the subject of a symposium in a special issue of *American Journal of Medicine*, July 1982.

Fluorinated carbohydrates have been studied before. A survey of the subject by Penglis is in *Advances in Carbohydrate Chemistry and Biochemistry 38*, 195—285 (1981). A 2,2-difluorohexose was described by Adamson et al., *Carbohydrate Research 18*, 345—47 (1971). Wright and Taylor, *Carbohydrate Research 6*, 347—54 (1968), teach the synthesis of 9-(3-deoxy-3-fluoro-α-D-arabinofuranosyl)adenine.

Recently the total synthesis of carbohydrates has become the subject of research, and a few papers have appeared. The synthesis requires stereospecific methods, and asymmetric epoxidation and asymmetric aldol reactions have been used successfully. Masamune, Sharpless et al., *J. Org. Chem. 47*, 1373——1 (1982).

Monofluorinated pyrinidine nucleosides exhibiting anti-viral effects are disclosed, inter alia, in EP—A—10205.

In accordance with the invention, nucleosides of formula (I):

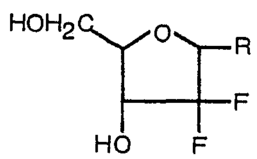

(I),

or a pharmaceutically-acceptable salt thereof, in which R is one of the following:

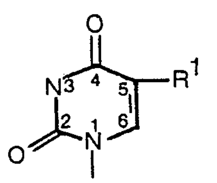

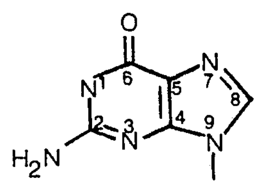

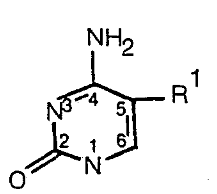

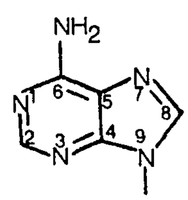

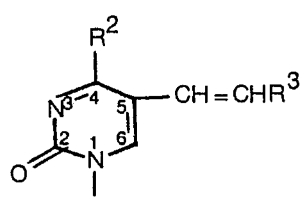

in which

$R^1$ is hydrogen, methyl, bromo, fluoro, chloro or iodo;

$R^2$ is hydroxy or amino;

$R^3$ is bromo, chloro or iodo, are useful antiviral agents.

In another aspect, a difluorodesoxy carbohydrate of formula (II):

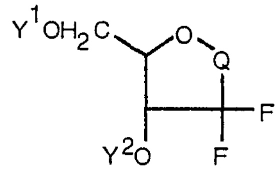

(II)

in which Q is CHOH, CHX in which X is a leaving group, or >C=O; and $Y^1$ and $Y^2$, independently, are hydrogen or hydroxy-protecting groups, is useful in the synthesis of antiviral agents of Formula (I).

In addition, the present invention provides a process for preparing a lactone of formula (III):

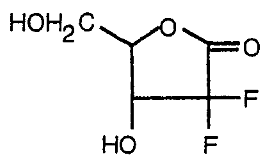

(III)

which process comprises hydrolyzing an alkyl 3-dioxolanyl-2,2-difluoro-3-hydroxypropionate of formula (IV):

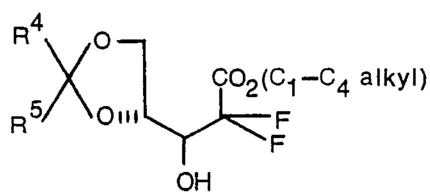

(IV)

in which $R^4$ and $R^5$ are, independently, $C_1$—$C_3$ alkyl.

Further, there is provided a process for preparing a compound of Formula (II) in which Q is CHOH, or a protected derivative thereof, which comprises reducing a compound of Formula (II) in which Q is >C=O, or a protective derivative thereof.

Throughout the specification, temperatures are described in degrees Celsius.

The structural drawings above do not indicate the stereochemistry of the compounds of the present invention. Compounds of all configurations are believed to be useful, and the stereochemistry of the compound is not to be construed as a limitation. The compound possessing the configuration of naturally occurring ribose, as follows, however, is preferred:

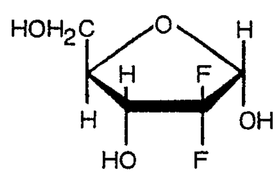

Further preferred is that the configuration of the juncture between the ribose and the base be as follows

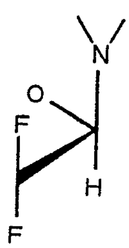

One skilled in the art would be aware of the bases which are used in the synthesis of the antiviral nucleosides of the present invention, but the following specific nucleosides are given to further elaborate the type of antiviral agents which this invention makes available.

1-(5-methyl-2,4-dioxo-1H,3H-pyrimidin-1-yl)-2-desoxy-2,2-difluororibose
1-(2,4-dioxo-1H,3H-pyrimidin-1-yl)-2-desoxy-2,2-difluororibose
1-(5-bromo-2,4-dioxo-1H,3H-pyrimidin-1-yl)-2-desoxy-2,2-difluororibose
1-(5-chloro-2,4-dioxo-1H,3H-pyrimidin-1-yl)-2-desoxy-2,2-difluororibose
1-(5-iodo-2,4-dioxo-1H,3H-pyrimidin-1-yl)-2-desoxy-2,2-difluororibose
1-(4-amino-5-chloro-2-oxo-1H-pyrimidin-1-yl)-2-desoxy-2,2-difluororibose
1-(4-amino-5-bromo-2-oxo-1H-pyrimidin-1-yl)-2-desoxy-2,2-difluororibose
1-(4-amino-2-oxo-1H-pyrimidin-1-yl)-2-desoxy-2,2-difluororibose
1-(4-amino-5-iodo-2-oxo-1H-pyrimidin-1-yl)-2-desoxy-2,2-difluororibose
1-(4-amino-5-methyl-2-oxo-1H-pyrimidin-1-yl)-2-desoxy-2,2-difluororibose
1-[5-(2-bromovinyl)-4-hydroxy-2-oxo-1H-pyrimidin-1-yl]-2-desoxy-2,2-difluororibose
1-[4-amino-5-(2-bromovinyl)-2-oxo-1H-pyrimidin-1-yl]-2-desoxy-2,2-difluororibose
1-[4-amino-5-(2-iodovinyl)-2-oxo-1H-pyrimidin-1-yl]-2-desoxy-2,2-difluororibose
1-[5-(2-chlorovinyl)-4-hydroxy-2-oxo-1H-pyrimidin-1-yl]-2-desoxy-2,2-difluororibose
1-[4-hydroxy-5-(2-iodovinyl)-2-oxo-1H-pyrimidin-1-yl]-2-desoxy-2,2-difluororibose
1-[4-amino-5-(2-chlorovinyl)-2-oxo-1H-pyrimidin-1-yl]-2-desoxy-2,2-difluororibose
1-(2-amino-6-oxo-1H,9H-purin-9-yl)-2-desoxy-2,2-difluoroibose
1-(6-amino-9H-purin-9-yl)-2-desoxy-2,2-difluororibose
1-(5-fluoro-2,4-dioxy-1H,3H-pyrimidin-1-yl)-2-desoxy-2,2-difluororibose
1-(2,4-dioxo-1H,3H-pyrimidin-1-yl)-2-desoxy-2,2-difluoroxylose
1-(5-bromo-2,4-dioxo-1H,3H-pyrimidin-1-yl)-2-desoxy-2,2-difluoroxylose
1-(5-chloro-2,4-dioxo-1H,3H-pyrimidin-1-yl)-2-desoxy-2,2-difluoroxylose
1-(5-iodo-2,4-dioxo-1H,3H-pyrimidin-1-yl)-2-desoxy-2,2-difluoroxylose
1-(4-amino-5-fluoro-2-oxo-1H-pyrimidin-1-yl)-2-desoxy-2,2-difluororibose
1-(4-amino-5-chloro-2-oxo-1H-pyrimidin-1-yl)-2-desoxy-2,2-difluoroxylose
1-(4-amino-2-oxo-1H-pyrimidin-1-yl)-2-desoxy-2,2-difluoroxylose
1-(4-aminp-5-fluoro-2-oxo-1H-pyrimidin-1-yl)-2-desoxy-2,2-difluoroxylose
1-(4-amino-5-methyl-2-oxo-1H-pyrimidin-1-yl)-2-desoxy-2,2-difluoroxylose
1-[5-(2-bromovinyl)-4-hydroxy-2-oxo-1H-pyrimidin-1-yl]-2-desoxy-2,2-difluoroxylose
1-[4-amino-5-(2-bromovinyl)-2-oxo-1H-pyrimidin-1-yl]-2-desoxy-2,2-difluoroxylose
1-[4-amino-5-(2-iodovinyl)-2-oxo-1H-pyrimidin-1-yl]-2-desoxy-2,2-difluoroxylose
1-[5-(2-chlorovinyl)-4-hydroxy-2-oxo-1H-pyrimidin-1-yl]-2-desoxy-2,2-difluoroxylose
1-[4-hydroxy-5-(2-iodovinyl)-2-oxo-1H-pyrimidin-1-yl]-2-desoxy-2,2-difluoroxylose
1-[4-amino-5-(2-chlorovinyl)-2-oxo-1H-pyrimidin-1-yl]-2-desoxy-2,2-difluoroxylose
1-(2-amino-6-oxo-1H,9H-purin-9-yl)-2-desoxy-2,2-difluoroxylose
1-(6-amino-9H-purin-9-yl)-2-desoxy-2,2-difluoroxylose, or the pharmaceutically-acceptable salts thereof.

The reactions of this invention in which the novel 2-desoxy-2,2-difluorocarbohydrate is coupled with the bases are frequently of a nature such that the hydroxy groups must be protected to keep them from reacting with other reagents present in the reaction. The protecting groups are those commonly used in synthetic organic chemistry. Chemists are accustomed to choosing groups which can be placed efficiently on hydroxy groups, and which can be removed easily when the reaction is complete. Suitable groups may be those described in standard textbooks, such as Chapter 3 of *Protective Groups in Organic Chemistry*, McOmie, Ed., Plenum Press, New York (1973); and Chapter 2 of *Protective Groups in Organic Synthesis*, Greene, John Wiley & Sons, New York (1981).

For example, hydroxy-protecting groups may include the formyl, 2-chloroacetyl, benzyl, diphenylmethyl, triphenylmethyl, 4-nitrobenzyl, phenoxycarbonyl, *t*-butyl, methoxymethyl, tetrahydropyranyl, allyl, tetrahydrothienyl, 2-methoxyethoxymethyl, methoxyacetyl, phenoxyacetyl, isobutyryl, ethoxycarbonyl, or benzyloxycarbonyl groups. Silyl hydroxy-protecting groups are particularly convenient because most of them are cleaved easily by contact with water or an alcohol. Such groups may include especially trimethylsilyl, as well as isopropyldimethylsilyl, methyldiisopropylsilyl, or triisopropylsilyl. The *t*-butyldimethylsilyl group is a special case and is preferred as the protecting group in this synthesis; it is more difficult to cleave, requiring a reagent such as a hydrohalic acid to remove it from the hydroxy groups.

Ribose or xylose has a hydroxy group at the 1-position of its ring. In order to react the carbohydrate of this invention with the base, to form the antiviral compounds of this invention, a leaving group must be placed at the 1-position. The leaving groups are those typically used in organic synthesis. The preferred leaving groups are sulfonates, of which the most preferred is methanesulfonate; other typical leaving groups such as toluenesulfonate, ethanesulfonate, isopropanesulfonate, 4-methoxybenzenesulfonate, 4-nitrobenzenesulfonate, 2-chlorobenzenesulfonate, chloro and bromo also may be used.

The following representative 2-desoxy-2,2-difluorocarbohydrates of the present invention are provided to further explain the invention:

2-desoxy-2,2-difluororibose
3,5-bis(trimethylsilyloxy)-2-desoxy-2,2-difluororibose
3,5-dibenzyloxy-2-desoxy-2,2-difluororibose

3,5-bis(chloroacetoxy)-2-desoxy-2,2-difluororibose
3,5-bis(2-chlorobenzyloxy)-1-methanesulfonyloxy-2-desoxy-2,2-difluororibose
3,5-bis(4-nitrobenzyloxy)-1-(4-toluenesulfonyloxy)-2-desoxy-2,2-difluororibose
1-chloro-3,5-bis(phenoxyacetoxy)-1,2-desoxy-2,2-difluoroxylose
1-(2,4-dibromophenylsulfonyloxy)-3,5-bis(2,2-dimethylpropionyloxy)-2-desoxy-2,2-difluoroxylose
3,5-bis(benzoyloxy)-1-(*o*-toluenesulfonyloxy)-2-desoxy-2,2-difluoroxylose
1-bromo-3,5-bis(methoxycarbonyloxy)-1,2-desoxy-2,2-difluoroxylose
3,5-bis(allyloxycarbonyloxy)-1-chloro-1,2-desoxy-2,2-difluoroxylose
3,5-bis(benzyloxycarbonyloxy)-2-desoxy-2,2-desoxy-2,2-difluoroxylose
1-bromo-3,5-bis(4-nitrobenzyloxycarbonyloxy)-1,2-desoxy-2,2-difluoroxylose
1-bromo-3,5-bis(tetrahydrothienyloxy)-1,2-desoxy-2,2-difluororibose
1-bromo-3,5-bis(isopropyldimethylsilyloxy)-1,2-desoxy-2,2-difluororibose
1-(2-chlorophenylsulfonyloxy)-3,5-bis(methoxymethoxy)-2-desoxy-2,2-difluororibose
3,5-bis(benzyloxymethoxy)-2-desoxy-2,2-difluororibose
1-(4-nitrophenylsulfonyloxy)-3,5-bis(trityloxy)-2-desoxy-2,2-difluororibose
3,5-bis(allyloxy)-1-chloro-1,2-desoxy-2,2-difluororibose
2-desoxy-2,2-difluoroxylose
3,5-bis(trimethylsilyloxy)-2-desoxy-2,2-difluoroxylose
3,5-dibenzyloxy-2-desoxy-2,2-difluoroxylose
3,5-bis(chloroacetoxy)-2-desoxy-2,2-desoxy-2,2-difluoroxylose
3,5-bis(2-chlorobenzyloxy)-1-methanesulfonyloxy-2-desoxy-2,2-difluoroxylose
3,5-bis(4-nitrobenzyloxy)-1-(4-toluenesulfonyloxy)-2-desoxy-2,2-difluoroxylose
1-bromo-3,5-bis(tetrahydrothienyloxy)-1,2-desoxy-2,2-difluoroxylose
1-bromo-3,5-bis(isopropyldimethylsilyloxy)-1,2-desoxy-2,2-difluoroxylose
3,5-bis(*t*-butyldiphenylsilyloxy)-2-desoxy-2,2-difluororibose
3,5-bis(formyloxy)-1-isopropylsulfonyloxy-2-desoxy-2,2-difluororibose
3,5-bis(trichloroacetoxy)-1-methanesulfonyloxy-2-desoxy-2,2-difluororibose
1-chloro-3,5-bis(phenoxyacetoxy)-1,2-desoxy-2,2-difluororibose
1-(2,4-dibromophenylsulfonyloxy)-3,5-bis(2,2-dimethylpropionyloxy)-2-desoxy-2,2-difluororibose
3,5-bis(benzoyloxy)-1-(*o*-toluenesulfonyloxy)-2-desoxy-2,2-difluororibose
1-bromo-3,5-bis(methoxycarbonyloxy)-1,2-desoxy-2,2-difluororibose
1-(2-chlorophenylsulfonyloxy)-3,5-bis(methoxymethoxy)-2-desoxy-2,2-difluoroxylose
3,5-bis(benzyloxymethoxy)-2-desoxy-2,2-difluoroxylose
1-(4-nitrophenylsulfonyloxy)-3,5-bis(trityloxy)-2-desoxy-2,2-difluoroxylose
3,5-bis(allyloxy)-1-chloro-1,2-desoxy-2,2-difluoroxylose
3,5-bis(*t*-butyldiphenylsilyloxy)-2-desoxy-2,2-difluoroxylose
3,5-bis(formyloxy)-1-isopropylsulfonyloxy-2-desoxy-2,2-difluoroxylose
3,5-bis(trichloroacetoxy)-1-methanesulfonyloxy-2-desoxy-2,2-difluoroxylose
3,5-bis(allyloxycarbonyloxy)-1-chloro-1,2-desoxy-2,2-difluororibose
3,5-bis(benzyloxycarbonyloxy)-2-desoxy-2,2-difluororibose
1-bromo-3,5-bis(4-nitrobenzyloxycarbonyloxy)-1,2-desoxy-2,2-difluororibose

The carbohydrates of the present invention are prepared by reacting a D-glyceraldehyde ketonide of formula (V):

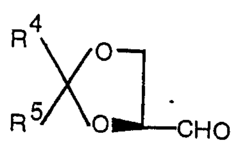

(V)

in which $R^4$ and $R^5$ are, independently, $C_1$—$C_3$ alkyl, with a $C_1$—$C_4$ alkyl bromodifluoroacetate, preferably the ethyl ester.

The preferred glyceraldehyde ketonide is the acetonide in which $R^4$ and $R^5$ are both methyl (see Fischer and Baer, *Helv. Chim. Acta. 17*, 622 (1934)). Ethyl bromodifluoroacetate was prepared first by Morel and Dawans, *Tet. 33*, 1445 (1977). The reaction of the ketonide and the haloacetate is carried out in the presence of an activated metal such as magnesium or preferably, zinc. Activation is obtained most easily by applying ultrasonic energy to the reaction mixture. Activation by that means compensates for the presence of a small amount of water in the reaction mixture, avoiding the necessity to maintain anhydrous conditions, and also avoids the necessity to prepare and carefully store activated metals. However, if desired, the metal may be activated by the customary methods known in the art. Approximately an equimolar amount of metal is the most advantageous amount.

The reaction has been performed in ethers such as tetrahydrofuran and diethyl ether, at moderate temperatures. However, other organic solvents which are inert to the reaction conditions may be used, including halogenated alkanes such as chloroform, dichloromethane, or trichloroethane, and aromatic solvents including benzene, toluene and the xylenes. Temperatures in the range of from ambient

temperature to 150° may be used; temperatures from ambient temperature to 80° are preferred, however. Economically-acceptable yields have been obtained in reaction times ranging from a few minutes to a few hours. One should note that the reaction is exothermic, and the mixture may need to be cooled, not heated, depending on the scale of the reaction and the rate at which the reactants are added.

The product of the first reaction is an alkyl 3-dioxolanyl-2,2-difluoro-3-hydroxypropionate of formula (IV):

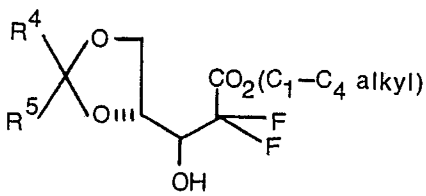

(IV)

in which $R^4$ and $R^5$ are as described earlier.

The ratio of the 3-R-hydroxy intermediate to its 3-S-hydroxy enantiomer is usually about 3:1. The 3-R-hydroxy enantiomer has the proper stereochemistry to produce the ribose derivative in its natural configuration, and so it is the desired enantiomeric product of the first step. The 3-R-hydroxy enantiomer can be separated cleanly from the 3-S-enantiomer by chromatography on silica gel, eluting with chloroform containing 0.5% methanol.

The hydroxypropionate, in either form, is hydrolyzed using very mild conditions to form the lactone of formula (III):

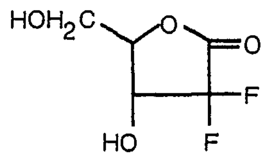

(III)

Proper control of the hydrolysis step will cleave the ketonide function and, exexpectedly, will also cleave the ester group, providing the lactone in a single step. The hydrolysis reagent preferably is a mildly acidic ion exchange resin, of which Dowex® 50W—X12 (Dow Chemical Company) is most highly preferred. Carrying out the process with other mild hydrolytic reagents is possible although larger amounts of byproducts may be obtained. For example, aqueous acetic acid, or other relatively strong acids such as propionic acid, formic acid, chloroacetic acid, or oxalic acid may be used for the hdyrolysis.

The hydroxy groups of the lactone should be protected before its keto oxygen is reduced. The usual reaction conditions are used, depending on the protecting groups chosen. For example, the *t*-butyldimethylsilyl group is most conveniently provided in the form of its trifluoromethanesulfonate, and the protection reaction is carried out in the presence of a base such as lutidine, pyridine and the like. Acyl protecting groups such as acetyl, benzoyl and the like are provided by reacting the lactone with an acylating agent such as an acyl chloride, bromide, cyanide or azide, or with an appropriate anhydride. The reactions are conveniently carried out in a basic solvent such as pyridine, quinoline or isoquinoline, or in a tertiary amine solvent such as triethylamine, tributylamine, or methylpiperidine. The reaction also may be carried out in an inert solvent, to which an acid scavenger, such as a tertiary amine, has been added. Acylation catalysts such as 4-dimethylaminopyridine or 4-pyrrolidinopyridine may be used in the reaction, if desired. The acylation reactions which provide protecting groups on the hydroxy groups are carried out at moderate temperatures in the range of from −25° to 100°. Such acylations also may be performed by acid-catalyzed reactions of the appropriate carboxylic acids, in inert organic solvents or neat. Acid catalysts such as sulfuric acid, polyphosphoric acid, or methanesulfonic acid may be used.

Acyl protecting groups may also be provided by forming an active ester of the appropriate acid, such as the esters formed by such known reagents as dicyclohexylcarbodiimide, acylimidazoles, nitrophenols, pentachlorophenol, N-hydroxysuccinimide and 1-hydroxybenzotriazole.

Protected groups of the ether type are produced by reacting the lactone with, for example, an appropriate diazo compound, such as diazomethane, phenyldiazomethane or a silyldiazomethane. Such reactions commonly are carried out in solvents including esters such as ethyl acetate, halogenated solvents including dichloromethane and chloroform, and ethers including diethyl ether and tetrahydrofuran. The process is usually carried out at low temperatures from −50° to 0°. Such ether-forming reactions may also be carried out with the assistance of reagents such as trimethyloxosulfonium hydroxide, trimethylsulfonium hydroxide and trimethylselenonium hydroxide, in solvents such as dimethylsulfoxide, dimethylformamide, hexamethylphosphoramide, acetone, or acetonitrile.

The silyl protecting groups discussed above are placed on the hydroxy groups by the conventional methods, such as by reaction with the appropriate silylcarboxamide or bis(substituted-silyl)carboxamide, or an appropriately substituted silazane. Suitably substituted silyl methanesulfonates, toluenesulfonates and the like are useful also. An equivalent amount of a base is usually necessary in the reaction mixture, unless a basic solvent such as is discussed above is used in the reaction.

When the hydroxy groups have been protected, the keto oxygen of the lactone is reduced to the alcohol, forming the protected 2-desoxy-2,2-difluororibose or xylose of this invention. The most preferred reducing agent is diisobutyl aluminum hydride, used at a low temperature in the range of about −100° to −20°. The reduction must be performed very carefully to avoid conditions so vigorous that the ring is opened at the oxygen atom. Other metal hydrides, such as the widely used lithium aluminum hydride, can also be used for the reduction, but it is necessary to keep the temperature quite low and to assure that the hydride is destroyed before the temperature is allowed to rise towards ambient. Accordingly, a solvent with a very low freezing point must be used in the reduction step. Toluene is convenient; other solvents, of course, can be used, including lower alkanols, especially ethanol, or ethers such as diethyl ether.

To obtain efficient reaction with the base, an appropriate leaving group must be placed at the 1-position of the carbohydrate. The preferred leaving group is methanesulfonyl, which is readily provided by reaction with methanesulfonyl chloride in the presence of an equivalent amount of a suitable acid scavenger such as triethylamine and the like. Other sulfonyl leaving groups are provided in the same way by reaction with the appropriate sulfonyl halide.

When a chloro or bromo leaving group is to be used, it is frequently advantageous first to make the l-acetate derivative, as by reaction with acetic anhydride, or another source of acetyl groups, in the presence of an equivalent amount or more of an acid scavenger. The acetate group then is displaced, at a low temperature such as −50° to 0°, with gaseous hydrogen bromide or hydrogen chloride. Because the gaseous hydrogen halide may tend to remove the protecting groups, especially silyl protecting groups, operating this step at low temperatures and adding the hydrogen halide slowly in small increments is necessary.

The bases used to form the antiviral compounds of the present invention are known to those skilled in the art, and no discussion of their synthesis is necessary. The primary amino groups present on some of the bases, however, should be protected before the base is coupled with the carbohydrate. The usual amino-protecting groups are used, including silyl groups such as have been discussed, as well as such typical groups as *t*-butoxycarbonyl, benzyloxycarbonyl, 4-methoxybenzyloxycarbonyl, 4-nitrobenzyloxycarbonyl, formyl, or acetyl.

Converting the keto oxygen atoms on the bases to the enol form, in order to make the base more highly aromatic and allowing a more ready attack of the base by the carbohydrate is advisable. Enolizization is provided most conveniently by producing the silyl protecting groups. The usual silyl protecting groups, as discussed above, may be used for this purpsoe.

The reaction between the protected carbohydrate and the base preferably is performed neat at an elevated temperature in the range of from 50° to 200°. Use of relatively high-boiling solvents for the reaction, such as dimethylformamide, dimethylacetamide, or hexamethylphosphoramide, however, is possible. If the coupling reaction is carried out at elevated pressures to avoid distillation of a low-boiling solvent, any convenient inert reaction solvent can be used.

The coupling reaction may be done at low temperatures if a reaction initiator, such as a trifluoromethanesulfonyloxysilane, is used. The usual inert reaction solvents, as discussed above, may be used at temperatures in the range of from about ambient temperature to about 100°.

The final step of the reaction sequence is the removal of the protecting groups. Most silyl protecting groups are cleaved easily by contact with water or an alcohol. The *t*-butyldimethylsilyl protecting group requires acid conditions, such as contact with gaseous hydrogen halide, for its removal.

Acyl protecting groups are removed by simple hydrolysis with strong or moderately strong bases, such as alkali metal hydroxides, at temperatures from about ambient temperature to about 100°. At least one equivalent amount of base is needed for each protecting group. Such hydrolyses conveniently are carried out in hydroxylic solvents, especially aqueous alkanols. The reactions also may be carried out, however, in any convenient solvent, such as polyols including ethylene glycol, ethers such as tetrahydrofuran, ketones such as acetone and methyl ethyl ketone and other polar solvents such as dimethylsulfoxide. The cleavage of acyl protecting groups may also be performed with other bases, including, for example, sodium methoxide, potassium t-butoxide, hydrazine, hydroxylamine, ammonia, alkali metal amides and secondary amines such as diethylamine. The acyl protecting groups also can be removed with acid catalysts, such as methanesulfonic acid, hydrochloric acid, hydrobromic acid, sulfuric acid, or the acidic ion exchange resins. Carrying out such hydrolyses at a relatively high temperature, such as the reflux temperature of the mixture is preferred, but temperatures as low as ambient may be used when particularly strong acids are used.

The removal of protecting groups which are ethers is carried out by known methods, for example, with ethanethiol and aluminum chloride.

None of the reaction steps require unusual excesses of the reactants. As usual in organic syntheses, use of a moderate excess, in the range of 1.05X to 2X, is advisable.

The antiviral compounds provided by this invention are capable of forming pharmaceutically-acceptable addition salts. Such salts are to be construed as included within the scope of this invention and may include hydrobromide, hydrochloride, mono-, di- or triphosphate esters and sodium salts of such phosphates, sulfate, the sodium, potassium, lithium or ammonium salts, as well as others well-known to those skilled in the art. "Pharmaceutically-acceptable salts" are those salts useful in the chemotherapy of warm-blooded animals.

The antiviral nucleosides of the present invention are used for the treatment of viral infections in the usual manner. The compounds are effective for the treatment of viral infections in general, and most particularly in the treatment of infections caused by viruses of the herpes genus.

The compounds provided by this invention, or a pharmaceutically-acceptable salt thereof, may be administered orally, topically or parenterally. In general, dosage rates in the range from 5 mg to 500 mg/kg are useful. It is more preferred to administer rates in the range of from 10 mg/kg to 100 mg/kg.

The compounds are useful usually in the form of pharmaceutical compositions. The formulation of the composions is conventional, and follows the usual practices of those skilled in the art. When a nucleoside of the present invention is to be administered topically, it is formulated as a topical composition, such as a cream or ointment to be rubbed into the affected tissue. Creams are emulsions of an oily phase and an aqueous phase, in which the nucleoside is dissolved or suspended. Ointments are greasy or waxy compositions, in which the nucleoside may be soluble but may be suspended, if it is insoluble at the desired concentration.

The terms "Pharmaceutically-acceptable" or "physiologically-acceptable" refer to those agents useful in the chemotherapy of warm-blooded animals.

Parenteral compositions are preferably formulated in such a way that the nucleoside, or pharmaceutically-acceptable salt thereof, can be dissolved for injection, but most of the nucleosides are not highly water-soluble. Thus, it is more common for a parenteral product to be formulated as a dried powder of the nucleoside and physiologically-acceptable suspending agents, such as starch and sugar, to which sterilized water is added to form a suspension to be injected. Parenteral compositions can be formulated in aqueous bases containing moderate amounts of physiologically-acceptable solvents, such as propylene glycol and the like, and such compositions may be capable of dissolving the present nucleosides at acceptable concentrations.

A great many types of orally administered compositions are in common use, including unit dosage forms such as tablets and capsules, and liquid dosage forms such as suspensions. In general, unit dosage forms are preferred in pharmacy and are formulated in such a way as to provide the usual dose in one or a small number of tablets or capsules. The formulation of tablets, making use of appropriate lubricants, binding agents and disintegration agents will be understood by those skilled in the art. The formulation of capsules involves only the dilution of the nucleoside with an appropriate proportion of an inert powdery substance, such as lactose, to provide the proper bulk to fill the desired size of capsule. The formulation of orally-administered suspensions is carried out by finely grinding the nucleoside, and intimately mixing it with a comparatively viscous aqueous-base liquid. The viscosity is adjusted by the addition of pharmaceutically-acceptable thickening or gel-forming agents including vegetable gums, chemically-modified cellulose derivatives and the like. Appropriate flavors may be used as desired.

The following non-limiting preparations, tests and examples are provided to illustrate further the invention.

## Preparation 1

Ethyl 2,2-difluoro-3-hydroxy-3-(2,2-dimethyldioxolan-4-yl)propionate

To 10.2 g of activated zinc was added a small portion of a solution consisting of 31.8 g of ethyl bromodifluoroacetate and 22.6 g of 4-formyl-2,2-dimethyldioxolane in 53 ml of tetrahydrofuran and 53 ml of diethylether. Care was taken to exclude water from the reaction mixture. The solution began to reflux as soon as the first addition to the activated zinc was made. The remainder of the solution was added dropwise at a rate to maintain gentle reflux throughout the addition time of about 30 minutes. The mixture was then stirred under gentle reflux for 30 minutes more. The reaction mixture was poured into 200 ml of 1N hydrochloric acid and 200 g of ice, and the mixture was stirred until all of the ice had melted. The aqueous mixture was then extracted four times with 70 ml portions of diethyl ether, and the organic layers were combined and washed with 50 ml of saturated aqueous sodium chloride and with 50 ml of saturated aqueous sodium bicarbonate, dried over magnesium sulfate and evaporated under vacuum to obtain 26 g of light yellow oil. The crude product was chromatographed on a 1000 g silica gel column, eluting with chloroform containing 0.5% methanol to separate the major 3-R-hydroxy product from the minor 3-S-hydroxy product. The ratio of amounts of the two products was about 3:1, the minor product came off the column first.

Evaporation of the fractions containing the 3-R-hydroxy product provided 12.6 g of the product in substantially pure form. It was identified by mass spectrometry, showing a fragment of weight 239, which agrees with the molecular weight of the desired product less a methyl group which was lost from the acetonide function in the spectrometric measurement. A nuclear magnetic resonance analysis of the 3-R-hydroxy product on a 90 mHz instrument in $CDCl_3$ showed $\delta$ = 3.94—4.45 (m, 5H); 3.14 (d, J = 4.5Hz, 1H); 1.2—1.47 (m, 9H).

Analysis by the same NMR procedure of the 3-S-hydroxy product, of which 4.68 g was obtained by evaporation of the appropriate chromatography fractions showed 3.75—4.47 (m, 6H); 2.95 (d, J = 8Hz, 1H); 1.25—1.5 (m, 9H).

Preparation 2

2-desoxy-2,2-difluoro-1-oxoribose

Fifty g of the 3-R-hydroxy product obtained from a synthesis similar to that of Preparation 1 above was dissolved in 500 ml of methanol and 250 ml of water, and 250 g of Dowex® 50W—X12 resin was added. The mixture was stirred at ambient temperature for 4 days, and the mixture was then filtered through a pad of diatomaceous earth filter aid. The filtrate was evaporated to dryness under vacuum to obtain 33.0 g of the desired product, which was identified by NMR analysis on a 90 mHz instrument in $CD_3OD$: δ = 3.6—4.6 (series of m, 4H); 4.8 (bs, 2H).

Preparation 3

3,5-bis(*t*-butyldimethylsilyloxy)-2-desoxy-2,2-difluoro-1-oxoribose

To 13 g of the product obtained in Preparation 2 above was added 60 ml of dichloromethane, 22.5 ml of 2,6-lutidine and 48.2 ml of trifluoromethylsulfonyloxy *t*-butyldimethylsilane under nitrogen with mild cooling to keep the temperature below 25°. Within 15 minutes after combining the reagents, the reaction became quite exothermic and the mixture became thin and easily stirred. The mixture was stirred overnight. The mixture was diluted with 150 ml of ethyl acetate, and was washed successively with 40 ml of 1N hydrochloric acid, 40 ml of saturated aqueous sodium bicarbonate and 40 ml of saturated aqueous sodium chloride. It was then dried over magnesium sulfate and evaporated to dryness under vacuum to obtain 32.1 g of crude product, which was chromatographed on 260 g of 100-mesh silica gel, eluting with 10:1 chloroform:diethyl ether. The fractions which contained the desired product were combined and evaporated under vacuum to obtain 7.8 g of pure product. Other fractions were combined and evaporated to obtain an additional 10 g of impure product, which was not further purified. Analysis of the pure product gave the following results: IR (neat) 1820 $cm^{-1}$; NMR ($CDCl_3$, 90 MHz) δ = .1—.22 (m, 12H); .83—.98 (m, 18H); 3.63—4.7 (series of m, 4H); Mass Spec. m/e = 339 = P-*t*-butyl.

Example 1

3,5-bis(*t*-butyldimethylsilyl)-2-desoxy-2,2-difluororibose

A 10.3 g portion of 3,5-bis(*t*-butyldimethylsilyloxy)-2-desoxy-2,2-difluoro-1-oxoribose, obtained from preparations similar to that of Preparation 3 above, was dissolved in 120 ml of anhydrous toluene and cooled to −84°. To the solution was added 26 g of diisobutyl aluminum hydride, added over a period of 20 minutes with constant stirring. The reaction mixture was held below −65° at all times. Two hours after the first addition of hydride, the reaction mixture was quenched with methanol at −20°, additional cold methanol was added until no more gassing occurred. The mixture was then allowed to warm slowly to ambient temperature, and was washed with 100 ml of 0.1N hydrochloric acid. The aqueous layer was then washed with 100 ml of diethyl ether, and then three times with 50 ml portions of diethyl ether. The organic layers were combined, washed with 100 ml of saturated aqueous sodium bicarbonate, dried over magnesium sulfate and evaporated under vacuum to dryness to obtain 8.2 g of the desired product in crude form.

This material may be chromatographed, if necessary, in silica gel (25 g silica/1 g of crude product) using 100% dichloromethane for elution. NMR ($CDCl_3$, 90 MHz) δ = .1—.24 (m, 12H); .85—1.0 (m, 18H); 3.33—4.63 (series of m, 5H); 5.0—5.27 (dd, 1H); Mass Spec. m/e = 341 = P-*t*-butyl; $[\alpha]_D^{25°}$ = +25.1°.

Example 2

3,5-bis(*t*-butyldimethylsilyloxy)-1-methane sulfonyloxy-2-desoxy-2,2-difluororibose

An 0.5 g portion of 3,5-bis(*t*-butyldimethylsilyloxy)-2-desoxy-2,2-difluororibose was dissolved in 5 ml of anhydrous dichloromethane and 0.17 g of triethylamine. To the solution was added, with mild cooling, 0.11 ml of methanesulfonyl chloride. After three hours of stirring under nitrogen at about 25°, the mixture was evaporated under vacuum, and the residue was taken up in 10 ml of ethyl acetate. The solution was extracted with 3 ml of saturated aqueous sodium bicarbonate, and then successively with 3 ml of 1N hydrochloric acid, 3 ml of water and 3 ml of saturated aqueous sodium chloride. The organic solution was then dried over sodium sulfate and concentrated under vacuum to obtain 0.59 g. of the desired product, NMR ($CDCl_3$, 90 MHz) δ .05—.16 (m, 12H); .78—.90 (m, 18H); 3.0 (s, 3H); 3.63—4.59 (series of m, 4H); 5.67—5.9 (dd, 1H); Mass Spec. m/e = 419 = P-*t*-butyl.

Example 3

1-(5-methyl-2,4-dioxo-1H,3H-pyrimidin-1-yl)-1,2-desoxy-2,2-difluororibose

Under nitrogen, to 2.59 g of 3,5-bis(*t*-butyldimethylsiloxy)-1-methanesulfonyloxy-2-desoxy-2,2-difluororibose was added 1.60 g of 5-methyl-2,4-bis(trimethylsilyloxy)pyrimidine and 45 ml of dry 1,2-dichloroethane. To this mixture was added 1.45 g of trifluoromethanesulfonyloxytrimethylsilane, and the clear solution was stirred under reflux for about 2—3 hours. The reaction was then cooled to ambient temperature and 1.35 ml of methanol were added and the suspension was stirred for 30 minutes. The precipitate was filtered and the filtrate was reduced to one-half its volume under vacuum and then diluted with an equal volume of dichloromethane. The solution was washed with saturated aqueous sodium bicarbonate and then with saturated aqueous sodium chloride, and dried over anhydrous sodium sulfate. The solution was filtered and the filtrate was saturated with anhydrous hydrogen bromide. The reaction

mixture was stirred for 30 minutes and was then concentrated under vacuum. The residue was dissolved in methanol and the solution was evaporated to dryness under vacuum. The residue was dissolved in water and the solution was extracted twice with diethyl ether. The water layer was then evaporated to dryness. The residue was taken up in ethanol and evaporated repeatedly to azeotrope off all water. One g of crude product was obtained, and was chromatographed on 30 g of Woelm silica gel (70—150 mesh), eluting with ethyl acetate to yield 0.76 g of desired product. It was further purified by recrystallization from ethyl acetate to obtain 0.37 g of white crystalline product. NMR ($CD_3OD$, 90 MHz) δ 1.93 (s, 3H); 3.5—4.67 (series of m, 4H); 4.83 (bs, 3H); 6.3 (t, J=9Hz, 1H); 7.47 (m, 1H); mass spec m/e = 278 = Parent.

Example 4

1-(4-amino-2-oxo-1H-pyrimidin-1-yl)-2-desoxy-2,2-difluororibose

Under a nitrogen atmosphere, to 5.0 g of 3,5-bis(t-butyldimethylsiloxy)-1-methanesulfonyloxy-2-desoxy-2,2-difluororibose in 100 ml of dry 1,2-dichloroethane was added 4.68 g of *bis*-trimethylsilyl N-acetylcytosine followed by 3.96 g of trifluoromethanesulfonyloxytrimethylsilane. The solution is refluxed for 3 to 15 hours. The reacton is cooled to room temperature, 2.0 ml of methanol are added and the suspension is stirred for about 30 minutes. The precipitate is filtered and the filtrate is concentrated *in vacuo*, to dryness. The residue is dissolved in methylene chloride, saturated with anhydrous HBr and stirred at room temperature about 45 minutes. The mixture is concentrated to dryness *in vacuo*, taken up into saturated methanolic ammonia and stirred about 15 hours at room temperature. The solution is concentrated to dryness *in vacuo*, triturated with $H_2O$, and the aqueous soluble portion is applied to a reversed phase silica gel column producing 100 mg of the desired product with eluted with water. NMR ($CD_3OD$, 90MHz) δ 3.7—4.65 (series of m, 4H), 4.83 (bs, 4H), 5.97 (d, J = 8Hz, 1H), 6.24 (t, J=8Hz, 1H), 7.88 (d, J=8Hz, 1H); Mass Spec. m/e = 263 = parent.

Example 5

1-(4-amino-5-iodo-2-oxo-1H-pyrimidin-1-yl)-2-desoxy-2,2-difluororibose

Under a nitrogen atmosphere, to 1.99 g of 3,5-bis(t-butyldimethylsiloxy)-1-methanesulfonyloxy-2-desoxy-2,2-difluororibose in 35 ml of dry 1,2-dichloroethane was added 2.08 g of *tris*-trimethylsilyl-5-iodocytosine followed by 1.11 g of trifluoromethanesulfonyloxytrimethylsilane. The solution is refluxed for 3 to 15 hours. The reaction is cooled to room temperature, 5.0 ml of methanol are added and the suspension is stirred for about 30 minutes. The precipitate is filtered and the filtrate is concentrated *in vacuo*, to dryness. The residue is dissolved in methylene chloride, saturated with anhydrous HBr and stirred at room temperature for about 45 minutes. The mixture is concentrated to dryness *in vacuo*. The residue is triturated with $H_2O$, neutralized with $NaHCO_3$ and separated on a reversed phase silica gel column ($H_2O$:MeOH(9:1)) to yield 26 mg. of the desired product. NMR ($CD_3OD$, 90MHz) δ 3.6—4.73 (series of m, 4H), 4.9 (bs, 4H), 6.25 (t, J=8Hz, 1H), 8.44 (s, 1H); Mass Spec. m/e=389=parent.

Example 6

1-(2,4-dioxo-5-fluoro-1H,3H-pyrimidin-1-yl)-2-desoxy-2,2-difuororibose

Under a nitrogen atmosphere, to 1.1 g of 3,5-bis(t-butyldimethylsiloxy)-1-methanesulfonyloxy-2-desoxy-2,2-difluororibose in 20 ml of dry 1,2-dichloroethane was added 2.83 g of bistrimethylsilyl-5-fluorouracil followed by 0.66 g of trifluoromethanesulfonyloxytrimethylsilane. The solution is refluxed for 3 to 15 hours. The reaction is cooled to room temperature, 1.0 ml of methanol is added and the suspension is stirred for about 30 minutes. The precipitate is filtered and the filtrate is concentrated *in vacuo* to dryness. The residue is dissolved in methylene chloride, saturated with anhydrous HBr and stirred at room temperature for about 45 minutes. The mixture is concentrated to dryness *in vacuo*. The residue is triturated with $H_2O$, neutralized with $NaHCO_3$ and separated on a reversed phase silica gel column using $H_2O$ as eluent to yield 30 mg of the desired product. NMR ($CD_3OD$, 90MHz) δ 3.5—4.5 (series of m, 4H), 4.65 (bs, 3H), 5.89 (t, J=8Hz, 1H), 7.94 (d, J=7Hz, 1H); Mass Spec. m/e=282= parent.

The antiviral effect of the compounds of this invention has been shown by a proven *in vacuo* test, which was carried out as follows.

Test 1

African green monkey kidney cells (BSC—1) or Hela cells grown in 25 $cm^2$ Falcon flasks at 37° in medium 199 with 5 percent inactivated fetal bovine serum (FBS), penicillin (150 units/ml) and streptomycin (150 μg/ml). When confluent monolayers were formed, the supernatent growth medium was removed and 0.3 ml of an appropriate dilution of the indicated virus was added to each flask. After adsorption for one hour at room temperature, the virus infected cell sheet was overlaid with a medium comprising one part 1 percent Ionagar No. 2 and one part double strength medium 199 with FCS (fetal calf serum), penicillin, and streptomycin and also containing the test compound at the indicated concentrations in micrograms per milliliter. A flask containing no test compound served as a control. The stock solutions of test compound were made up in dimethylsulfoxide at a concentration of $10^4$μg/ml. The flasks were incubated for 72 hours at 37°. Plaques were seen in those areas where the virus infected and reproduced in the cells. A solution of 10 percent formalin and 2 percent sodium acetate was added to each flask to inactivate the virus and fix the cell sheet to the surface of the flask. The virus plaques, irrespective of size, were counted after staining the surrounding cell areas with crystal violet. The plaque count was compared to the control count at each drug concentration. The activity of the compound was expressed as percentage plaque inhibition.

The results of these evaluations are reported in Tables 1 to 6.

TABLE 1
Percent Plaque Inhibition at Specified µg/ml of Compounds
in Agar Overlay Using BSC—1 Cells
*Herpes simplex*, type I

| Compound of Example | 100 | 50 | 25 | 20 | 12.5 | 10 | 6.25 | 3.12 | 2 | 1.56 | 1 | 0.78 | 0.39 | .2 | |
|---|---|---|---|---|---|---|---|---|---|---|---|---|---|---|---|
| 3[a] | 96% | 72% | 53% | — | 35% | — | 15% | 12% | — | 0 | — | 8% | | 4% | — |
| 3[b] | 99% | — | — | 99% | — | 98% | — | — | 21% | — | 31% | — | — | — | |
| 4 | — | — | — | 100% | — | 100% | — | — | 100% | — | 100% | — | — | 100% | |

[a]anomeric mixture (α and β configurations)
[b]β-configuration anomer only

TABLE 2
Percent Plaque Inhibition at Specified μg/ml
of Compounds in Agar Overlay Using BSC—1 Cells
*Pseudorabies Virus*

| Compound of Example | 100 | 50 | 25 | 20 | 12 | 10 | 6 | 3 | 2 | 1 | .2 | .1 |
|---|---|---|---|---|---|---|---|---|---|---|---|---|
| 3[a] | 23% | 18% | 15% | — | 12% | — | 6% | 5% | — | — | — | — |
| 4 | — | — | — | 100% | — | 100% | — | — | 100% | 100% | 100% | 97% |

[a]anomeric mixture (α and β configurations)

TABLE 3
Percent Plaque Inhibition at Specified μg/ml
of Compounds in Agar Overlay Using BSC—1 Cells
*Herpes simplex*, Type II

| Compound of Example | 100 | 20 | 10 | 2 | 1 |
|---|---|---|---|---|---|
| 3[a] | — | 100% | 100% | 83 | 28 |

[a]β-configuration anomer only

TABLE 4

Percentıque Inhibition at Specified μg/ml
of Compounds in Agar Overlay Using BSC—1 Cells
*Polio virus*, Type I

| Compound of Example | 100 | 20 | 10 | 2 | 1 | .2 |
|---|---|---|---|---|---|---|
| 4 | — | — | 100% | 100% | 100% | — |

TABLE 5
Percent Plaque Inhibition at Specified
μg/ml of Compounds in Agar Overlay Using Hela Cells
*Herpes Simplex*, Type II

| Compound of Example | 100 | 20 | 10 | 2 | 1 | |
|---|---|---|---|---|---|---|
| 3[a] | 100% | 100% | 100% | 99% | 80% | |
| 3[b] | 65 | — | 7% | | — | — |

[a]β-configuration anomer
[b]α-configuration anomer

TABLE 6
Percent Plaque Inhibition at Specified
μg/ml of Compounds in Agar Overlay Using Hela Cells
*Herpes Simplex*, Type I

| Compound of Example | 100 | 20 | 10 | 2 | 1 | .2 |
|---|---|---|---|---|---|---|
| 3[a] | — | 100% | 100% | 99% | 99% | 42% |
| 4 | — | 100%[b] | 100%[b] | 100% | 98% | 54% |

[a]β-anomer only
[b]Possibly Toxic

Table 7 compares other known antiviral agents to the antiviral agents of the present invention. In particular, the compound of Example 3 is compared to Ara-A and Acyclovir for the dosage required to reduce by 50% the growth of *Herpes simplex*, type 1. This dosage is designated as the $ID_{50}$.

TABLE 7
Inhibition ($ID_{50}$) of Herpes simplex, type I
by Various Antiviral Agents

| Compound | $ID_{50}$ |
|---|---|
| Example 3[a] | .31 |
| Ara-A | 7.6 |
| Acyclovir | 1.74 |

[a]β-anomer only

Some of the compounds provided by the invention have been evaluated in an *in vitro* tissue culture screen. This assay involves the growth of a sheet of cells on the bottom of a tissue culture plate. The cells were infected with a virus strain and overlayed with a uniform layer of nutrient agar. Filter paper discs impregnated with measured amounts of test compound were then applied on the surface of the agar. The plates were incubated at 37°C and the cells are then fixed with Formalin and strained with a tetrachrome strain. Zones of antiviral activity attributable to the test compound are then read in millimeters. The morphology of protected cells is evaluated on a scale of 0 to 4, with 0 indicating completely damaged cells and 4 indicating normal cells.

Table 8 presents the results of this assay. Compounds were analyzed at the indicated concentrations. Zone sizes of cell protection are given in millimeters. The numbers in parenthesis following the zone sizes are the morphology readings.

TABLE 8

*In vitro* Tissue Culture Screen

| Compound from Example | Concentration impregnating solution (μg/ml) | Virus Strains Used | | | |
|---|---|---|---|---|---|
| | | Polio III | Herpes | Ann Arbor | Semiliki Forest |
| 4 | 5000 | – | 65 (4) | — | — |
| | 1000 | 47 (4) | 50 (4) | 45 (4) | 19 (4) |
| | 500 | 44 (4) | 45 (4) | 40 (4) | 17 (4) |
| | 250 | 39 (4) | 40 (4) | 32 (4) | 14 (4) |
| | 125 | 34 (4) | 37 (4) | 24 (4) | 10 (4) |
| | 62.5 | 28 (4) | 34 (4) | 21 (4) | 7 (3) |
| | 31.25 | 15 (4) | 30 (4) | 16 (4) | — |
| | 25 | 15 (4) | 27 (4) | — | |
| | 12.5 | 14 (3) | 24 (4) | — | — |
| | 6.25 | 9 (1) | 20 (4) | — | — |
| | 3.13 | — | 15 (4) | — | — |
| | 1.56 | — | 9 (4) | — | — |
| | .78 | — | 7 (2) | — | — |

**Claims for the Contracting States: BE CH DE FR GB IT LI LU NL SE**

1. A nucleoside of formula (I)

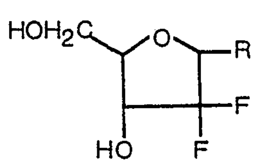

(I),

a protected derivative thereof, or a pharmaceutically-acceptable salt thereof, in which R is a base of one of the formulae:

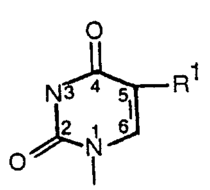

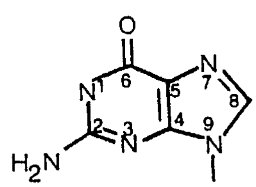

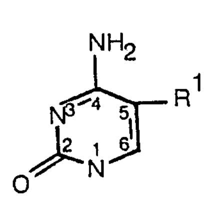

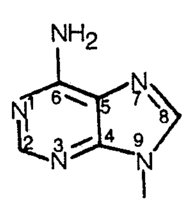

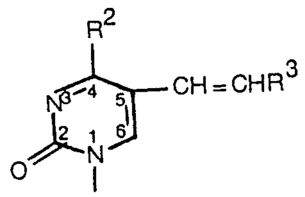

in which
$R^1$ is hydrogen, methyl, bromo, fluoro, chloro or iodo;
$R^2$ is hydroxy or amino;
$R^3$ is bromo, chloro or iodo.

2. A nucleoside as claimed in claim 1 in which the carbohydrate moiety is 2-desoxy-2,2-difluororibosyl.

3. A nucleoside as claimed in claim 1 or claim 2, or a pharmaceutically-acceptable salt thereof, in which R is

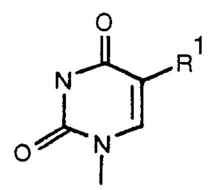

or

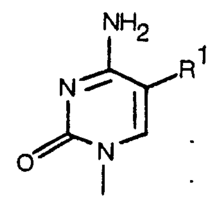

in which
$R^1$ is as defined in claim 1.

4. A nucleoside as claimed in any one of claims 1 to 3, or a pharmaceutically-acceptable salt thereof in which R is

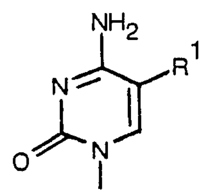

and $R^1$ is as defined in claim 1.

5. 1 - (5 - methyl - 2,4 - dioxo - 1H,3H - pyrimidin - 1 - yl) - 1,2 - desoxy - 2,2 - difluororibose, or a pharmaceutically-acceptable salt thereof.

6. 1 - (4 - amino - 2 - oxo - 1H - pyrimidin - 1 - yl) - 2 - desoxy - 2,2 - difluororibose, or a pharmaceutically-acceptable salt thereof.

7. 1 - (4 - amino - 5 - iodo - 2 - oxo - 1H - pyrimidin - 1 - yl) - 2 - desoxy - 2,2 - difluororibose, or a pharmaceutically-acceptable salt thereof.

8. 1 - (2,4 - dioxo - 5 - fluoro - 1H,3H - pyrimidin - 1 - yl) - 2 - desoxy - 2,2 - difluororibose, or a pharmaceutically-acceptable salt thereof.

9. A difluoro-desoxy carbohydrate of formula (II):

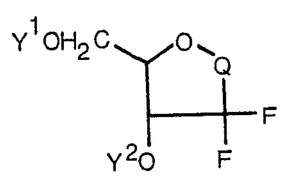

(II)

in which Q is CHOH, CHX in which X is a leaving group, or >C=O; and $Y^1$ and $Y^2$, independently, are hydrogen or hydroxy-protecting groups.

10. A carbohydrate as claimed in claim 9 in which Q is CHOH.

11. A carbohydrate as claimed in claim 9 or 10 in which the carbohydrate possesses the same configuration as ribose.

12. A carbohydrate as claimed in claim 9 in which Q is >C=O.

13. A carbohydrate as claimed in claim 9 in which X or CHX is a solfonate leaving group, chloro or bromo.

14. A carbohydrate as claimed in any one of claims 9 to 13 in which $Y^1$ and $Y^2$ are hydrogen, or a protected derivative thereof.

15. 2-Desoxy-2,2-difluoro-2-oxo-ribose, or a protected derivative thereof.

16. 1-Methanesulfonyloxy-2-desoxy-2,2-difluororibose, or a protected derivative thereof.

17. 2-Desoxy-2,2-difluororibose, or a protected derivative thereof.

18. A process for preparing a compound of Formula (I) as defined in claims 1 to 8 which comprises the removal of the protecting groups from a correspondingly protected nucleoside of Formula (I).

19. A process for preparing a compound of Formula (I) as defined in claims 1 to 8 which comprises coupling a compound of Formula (II), as defined in claim 9, in which Q is CHX, and $Y^1$ and $Y^2$ are hydroxy, or a protected derivative thereof, with a base as defined in claim 1, and, if desired, removing the protecting groups.

20. A process for preparing a compound of Formula (II), as defined in claim 9, in which Q is CHOH, or a protected derivative thereof, which comprises reducing a compound of Formula (II), in which Q is >C=O, or a protected derivative thereof.

21. A nucleoside of formula (I), or a pharmaceutically-acceptable salt thereof, as claimed in any one of claims 1 to 8, for use as an antiviral agent.

22. A nucleoside of formula (I), or a pharmaceutically-acceptable salt thereof, as claimed in any one of claims 1 to 8, for use in the treatment of herpes infections.

23. A pharmaceutical formulation which comprises as an active ingredient, a nucleoside of formula (I), or a pharmaceutically acceptable salt thereof, as claimed in any one of claims 1 to 8, associated with one or more physiologically-acceptable carriers or vehicles therefor.

**Claims for the Contracting State: AT**

1. A process for preparing a nucleoside of formula (I)

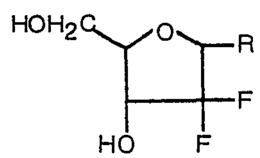

(I),

a protected derivative thereof, or a pharmaceutically-acceptable salt thereof, in which R is a base of one of the formulae:

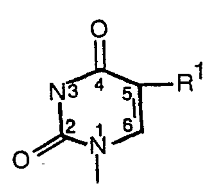

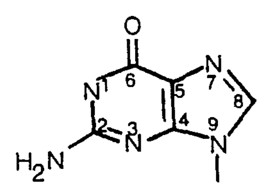

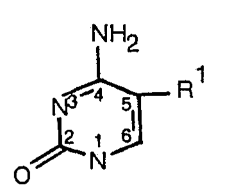

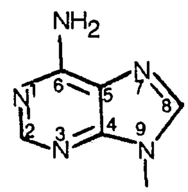

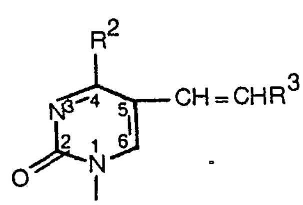

in which

$R^1$ is hydrogen, methyl, bromo, fluoro, chloro or iodo;

$R^2$ is hydroxy or amino;

$R^3$ is bromo, chloro or iodo, which comprises the removal of the protecting groups from a correspondingly protected nucleoside of Formula (I).

2. A process for preparing a compound of Formula (I), or a pharmaceutically-acceptable salt thereof, as defined in claim 1, which comprises coupling a compound of Formula (II):

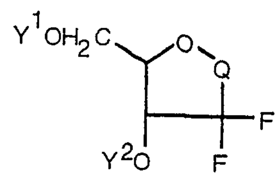

( II )

in which Q is CHX in which X is a leaving group, and $Y^1$ and $Y^2$, independently, are hydrogen or hydroxy-protecting groups, with a base as defined in claim 1, and, if desired, removing any protecting groups.

3. A process for preparing a nucleoside as claimed in claim 1 or 2 in which the carbohydrate moiety is 2-desoxy-2,2-difluororibosyl.

4. A process for preparing a nucleoside as claimed in any of claims 1 to 3 or a pharmaceutically-acceptable salt thereof, in which R is

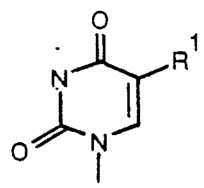

or

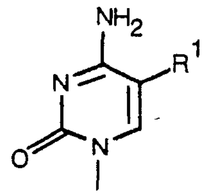

in which $R^1$ is as defined in claim 1.

5. A process for preparing a nucleoside as claimed in any one of claims 1 to 4, or a pharmaceutically-acceptable salt thereof in which R is

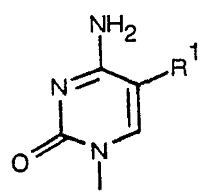

and $R^1$ is as defined in claim 1.

6. A process as claimed in any one of claims 1 to 4 for preparing 1 - (5 - methyl - 2,4 - dioxo - 1H,3H - pyrimidin - 1 - yl) - 1,2 - desoxy - 2,2 - difluororibose, or a pharmaceutically-acceptable salt thereof.

7. A process as claimed in any one of claims 1 to 5 for preparing 1 - (4 - amino - 2 - oxo - 1H - pyrimidin - 1 - yl) - 2 - desoxy - 2,2 - difluororibose, or a pharmaceutically-acceptable salt thereof.

8. A process as claimed in any one of claims 1 to 5 for preparing 1 - (4 - amino - 5 - iodo - 2 - oxo - 1H - pyrimidin - 1 - yl) - 2 - desoxy - 2,2 - difluororibose, or a pharmaceutically-acceptable salt thereof.

9. A process as claimed in any one of claims 1 to 4 for preparing 1 - (2,4 - dioxo - 5 - fluoro - 1H,3H - pyrimidin - 1 - yl) - 2 - desoxy - 2,2 - difluororibose, or a pharmaceutically-acceptable salt thereof.

10. A process for preparing a compound of Formula (II):

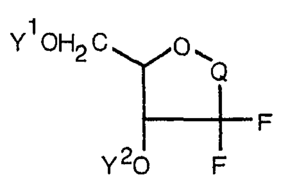

( II )

in which Q is CHOH, and $Y^1$ and $Y^2$ are, independently, hydrogen or hydroxy-protecting groups, which comprises reducing a compound of Formula (II) in which Q is >C=O, or a protected derivative thereof, and if desired, removing any protecting group present.

11. A process for preparing a compound of Formula (II), in which Q is CHOH, as claimed in claim 10, in which the product possesses the same configuration as ribose.

12. A process for preparing 2-desoxy-2,2-difluoro ribose, or protected derivative thereof as claimed in claim 10 or 11.

13. A nucleoside of Formula (I), or a pharmaceutically-acceptable salt thereof, whenever prepared by a process according to any one of claims 1 to 9.

14. A carbohydrate of Formula (II), or a protected derivative thereof, whenever prepared by a process according to any one of claims 10 to 12.

**Patentansprüche für die Vertragsstaaten: BE CH DE FR GB IT LI LU NL SE**

1. Nucleosid der Formel (I)

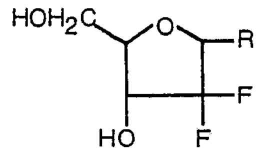

(I),

oder ein geschütztes Derivat hiervon oder ein pharmazeutisch annehmbares Salz hiervon, worin R irgendeine Base der folgenden Formeln ist:

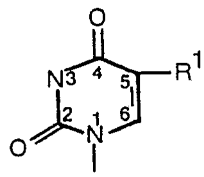

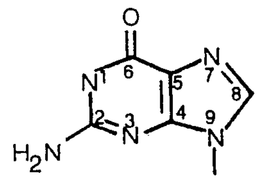

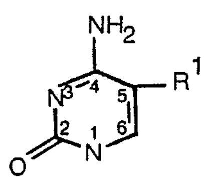

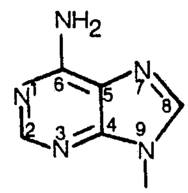

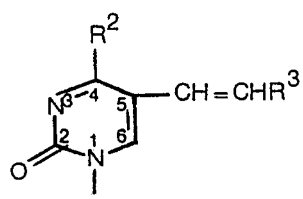

wobei
$R^1$ Wasserstoff, Methyl, Brom, Fluor, Chlor oder Iod ist,
$R^2$ Hydroxy oder Amino darstellt und
$R^3$ für Brom, Chlor oder Iod steht.

2. Nucleosid nach Anspruch 1, worin der Kohlenhydrat-2-Desoxy-2,2-difluorribosyl ist.

3. Nucleosid nach Anspruch 1 oder 2 oder ein pharmazeutisch annehmbares Salz hiervon, worin R für

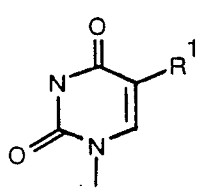

oder

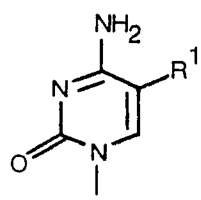

steht, wobei $R^1$ wie im Anspruch 1 definiert ist.

4. Nucleosid nach irgendeinem der Ansprüche 1 bis 3 oder ein pharmazeutisch annehmbares Salz hiervon, worin R für

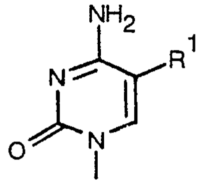

steht und $R^1$ wie im Anspruch 1 definiert ist.

5. 1-(5-Methyl-2,4-dioxo-1H,3H-pyrimidin-1-yl)-1,2-desoxy-2,2-difluorribose oder ein pharmazeutisch annehmbares Salz hiervon.

6. 1-(4-Amino-2-oxo-1H-pyrimidin-1-yl)-2-desoxy-2,2-difluorribose oder ein pharmazeutisch annehmbares Salz hiervon.

7. 1-(4-Amino-5-iod-2-oxo-1H-pyrimidin-1-yl)-2-desoxy-2,2-difluorribose oder ein pharmazeutisch annehmbares Salz hiervon.

8. 1-(2,4-Dioxo-5-fluor-1H,3H-pyrimidin-1-yl)-2-desoxy-2,2-difluorribose oder ein pharmazeutisch annehmbares Salz hiervon.

9. Difluordesoxykohlenhydrat der Formel (II)

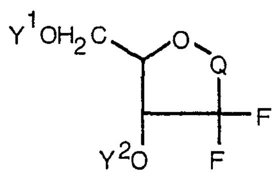

(II)

worin Q für CHOH, CHX, wobei X eine Abgangsgruppe ist, oder eine Gruppe =C=O steht, und $Y^1$ sowie $Y^2$ unabhängig Wasserstoff oder Hydroxyschutzgruppen sind.

10. Kohlenhydrat nach Anspruch 9, dadurch gekennzeichnet, daß Q für CHOH steht.

11. Kohlenhydrat nach Anspruch 9 oder 10, dadurch gekennzeichnet, daß es die gleiche Konfiguration wie Ribose hat.

12. Kohlenhydrat nach Anspruch 9, dadurch gekennzeichnet, daß Q für =C=O steht.

13. Kohlenhydrat nach Anspruch 9, dadurch gekennzeichnet, daß X von CHX eine Sulfonatabgangsgruppe, Chlor oder Brom ist.

14. Kohlenhydrat nach irgendeinem der Ansprüche 9 bis 13, dadurch gekennzeichnet, daß $Y^1$ und $Y^2$ Wasserstoff sind, oder daß es sich um ein geschütztes Derivat hiervon handelt.

15. 2-Desoxy-2,2-difluor-1-oxoribose oder ein geschütztes Derivat hiervon.

16. 1-Methansulfonyloxy-2-desoxy-2,2-difluorribose oder ein geschütztes Derivat hiervon.

17. 2-Desoxy-2,2-difluorribose oder ein geschütztes Derivat hiervon.

18. Verfahren zur Herstellung einer Verbindung der Formel (I) gemäß Anspruch 1 bis 8, dadurch gekennzeichnet, daß man von einem entsprechend geschützten Nucleosid der Formel (I) die Schutzgruppen entfernt.

19. Verfahren zur Herstellung einer Verbindung der Formel (I) gemäß Anspruch 1 bis 8, dadurch gekennzeichnet, daß man eine Verbindung der Formel (II) gemäß Definition von Anspruch 9, worin Q für CHX steht und $Y^1$ sowie $Y^2$ Hydroxy sind, oder ein geschütztes Derivat hiervon mit einer Base gemäß Definition von Anspruch 1 kuppelt und gewünschtenfalls die Schutzgruppen entfernt.

20. Verfahren zur Herstellung einer Verbindung der Formel (II) gemäß Definition von Anspruch 9, worin Q für CHOH steht, oder eines geschützten Derivats hiervon, dadurch gekennzeichnet, daß man eine Verbindung der Formel (II), worin Q für =C=O steht, oder ein geschütztes Derivat hiervon reduziert.

21. Verwendung eines Nucleosids der Formel (I) oder eines pharmazeutisch annehmbares Salzes hiervon gemäß irgendeinem der Ansprüche 1 bis 8 als antivirales Mittel.

22. Verwendung eines Nucleosids der Formel (I) oder eines pharmazeutisch annehmbares Salzes hiervon gemäß irgendeinem der Ansprüche 1 bis 8 zur Behandlung von Herpesinfektionen.

23. Pharmazeutische Formulierung, dadurch gekennzeichnet, daß sie ein Nucleosid der Formel (I) oder ein pharmazeutisch annèhmbares Salz hiervon gemäß irgendeinem der Ansprüche 1 bis 8 als Wirkstoff in Verbindung mit einem oder mehreren physiologisch annehmbaren Trägern oder Schleppmitteln hierfür enthält.

**Patentansprüche für den Vertragsstaat: AT**

1. Verfahren zur Herstellung eines Nucleosids der Formel (I)

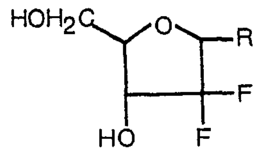

(I),

oder eines geschützten Derivats hiervon oder einen pharmazeutisch annehmbaren Salzes hiervon, worin R irgendeine Base der folgenden Formeln ist:

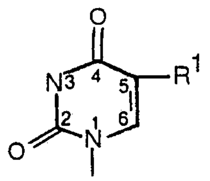

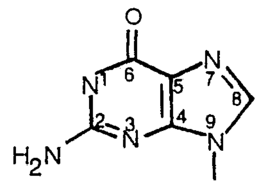

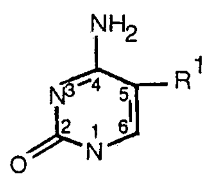

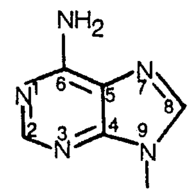

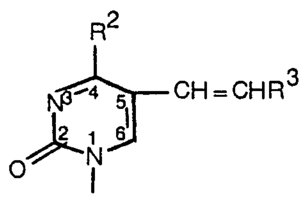

wobei

$R^1$ Wasserstoff, Methyl, Brom, Fluor, Chlor oder Iod ist,

$R^2$ Hydroxy oder Amino darstellt und

$R^3$ für Brom, Chlor oder Iod steht,

dadurch gekennzeichnet, daß man von einem entsprechend geschützten Nucleosid der Formel (I) die Schutzgruppen entfernt.

2. Verfahren zur Herstellung einer Verbindung der Formel (I) oder eines pharmazeutisch annehmbaren Salzes hiervon gemäß Anspruch 1, dadurch gekennzeichnet, daß man eine Verbindung der Formel (II)

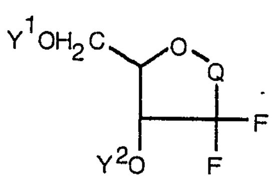

(II)

worin Q für CHX steht und $Y^1$ sowie $Y^2$ Hydroxy sind, oder ein geschütztes Derivat hiervon mit einer Base gemäß Definition von Anspruch 1 kuppelt und gewünschtenfalls die Schutzgruppen entfernt.

3. Verfahren zur Herstellung eines Nucleosids gemäß Anspruch 1 oder 2, dadurch gekennzeichnet, daß der Kohlenhydratrest 2-Desoxy-2,2-difluorribosyl ist.

4. Verfahren zur Herstellung eines Nucleosids gemäß irgendeinem der Ansprüche 1 bis 3 oder eines pharmazeutisch annehmbaren Salzes hiervon, dadurch gekennzeichnet, daß R für

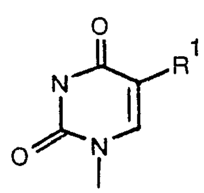

oder

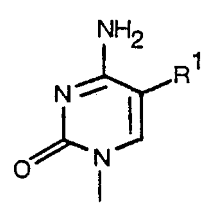

steht, worin $R^1$ wie im Anspruch 1 definiert ist.

5. Verfahren zur Herstellung eines Nucleosids gemäß irgendeinem der Ansprüche 1 bis 4 oder eines pharmazeutisch annehmbares Salzes hiervon, dadurch gekennzeichnet, daß R für

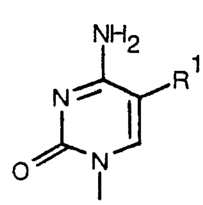

steht und $R^1$ wie im Anspruch 1 definiert ist.

6. Verfahren nach irgendeinem der Ansprüche 1 bis 4, dadurch gekennzeichnet, daß man 1-(5-Methyl-2,4-dioxo-1H,3H-pyrimidin-1-yl)-1,2-desoxy-2,2-difluorribose oder ein pharmazeutisch annehmbares Salz hiervon herstellt.

7. Verfahren nach irgendeinem der Ansprüche 1 bis 5, dadurch gekennzeichnet, daß man 1-(4-Amino-2-oxo-1H-pyrimidin-1-yl)-2-desoxy-2,2-difluorribose oder ein pharmazeutisch annehmbares Salz hiervon herstellt.

8. Verfahren nach irgendeinem der Ansprüche 1 bis 5, dadurch gekennzeichnet, daß man 1-(4-Amino-5-iod-2-oxo-1H-pyrimidin-1-yl)-2-desoxy-2,2-difluorribose oder ein pharmazeutisch annehmbares Salz hiervon herstellt.

9. Verfahren nach irgendeinem der Ansprüche 1 bis 4, dadurch gekennzeichnet, daß man 1-(2,4-Dioxo-5-fluor-1H,3H-pyrimidin-1-yl)-2-desoxy-2,2-difluorribose oder ein pharmazeutisch annehmbares Salz hiervon herstellt.

10. Verfahren zur Herstellung einer Verbindung der Formel (II)

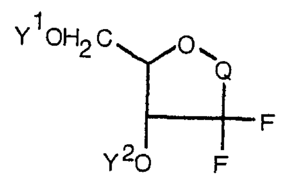

(II)

worin Q für CHOH steht und $Y^1$ sowie $Y^2$ unabhängig Wasserstoff oder Hydroxyschutzgruppen sind, dadurch gekennzeichnet, daß man eine Verbindung der Formel (II), worin Q für =C=O steht, oder ein geschütztes Derivat hiervon reduziert und gewünschtenfalls irgendeine vorhandene Schutzgruppe entfernt.

11. Verfahren zur Herstellung einer Verbindung der Formel (II), worin Q für CHOH steht, gemäß Anspruch 10, dadurch gekennzeichnet, daß das Produkt die gleiche Konfiguration wie Ribose hat.

12. Verfahren zur Herstellung von 2-Desoxy-2,2-difluorribose oder eines geschützten Derivats hiervon gemäß Anspruch 10 oder 11.

13. Nucleosid der Formel (I) oder ein pharmazeutisch annehmbares Salz hiervon, dadurch gekennzeichnet, daß es nach einem Verfahren irgendeines der Ansprüche 1 bis 9 hergestellt worden ist.

14. Kohlenhydrat der Formel (II) oder ein geschütztes Derivat hiervon, dadurch gekennzeichnet, daß es nach einem Verfahren irgendeines der Ansprüche 10 bis 12 hergestellt worden ist.

**Revendications pour les Etats contractants: BE CH DE FR GB IT LI LU NL SE**

1. Nucléoside de formule (I):

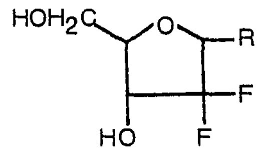

(I),

un de ses dérivés protégés ou un de ses sels pharmaceutiquement acceptables, formule dans laquelle R représente une base répondant à une des formules suivantes:

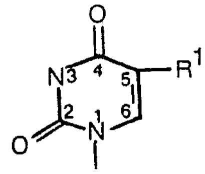

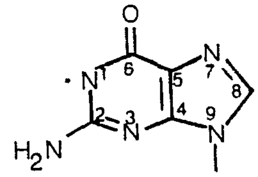

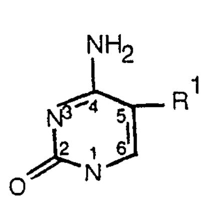

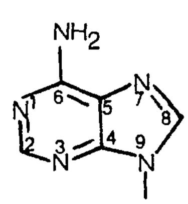

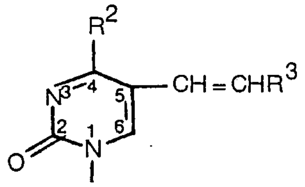

où

$R^1$ représente un atome d'hydrogène, un groupe méthyle, un atome de brome, un atome de fluor, un atome de chlore ou un atome d'iode;

$R^2$ représente un groupe hydroxyle ou un groupe amino;

$R^3$ représente un atome de brome, un atome de chlore ou un atome d'iode.

2. Nucléoside selon la revendication 1, dans lequel la fraction carbohydrate est le groupe 2-désoxy-2,2-difluororibosyle.

3. Nucléoside selon la revendication 1 ou 2, ou un de ses sels pharmaceutiquement acceptables, dans lequel R représente

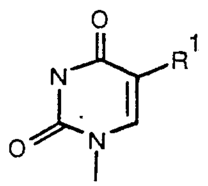

ou

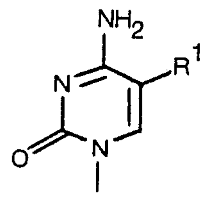

où

$R^1$ a la signification définie dans la revendication 1.

4. Nucléoside selon l'une quelconque des revendications 1 à 3, ou un de ses sels pharmaceutiquement acceptables, dans lequel R représente

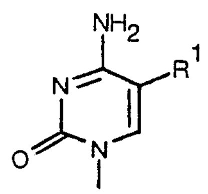

et $R^1$ a la signification définie dans la revendication 1.

5. Le 1-(5-méthyl-2,4-dioxo-1H,3H-pyrimidin-1-yl)-1,2-désoxy-2,2-difluororibose ou un de ses sels pharmaceutiquement acceptables.

6. Le 1-(4-amino-2-oxo-1H-pyrimidin-1-yl)-2-désoxy-2,2-difluororibose ou un de ses sels pharmaceutiquement acceptables.

7. Le 1-(4-amino-5-iodo-2-oxo-1H-pyrimidin-1-yl)-2-désoxy-2,2-difluororibose ou un de ses sels pharmaceutiquement acceptables.

8. Le 1-(2,4-dioxo-5-fluoro-1H,3H-pyrimidin-1-yl)-2-désoxy-2,2-difluororibose ou un de ses sels pharmaceutiquement acceptables.

9. Difluoro-désoxy-carbohydrate de formule (II):

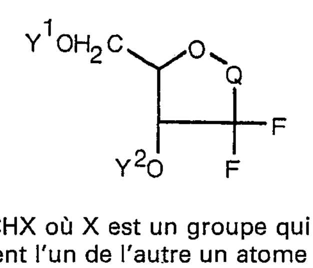

(II)

dans laquelle Q représente CHOH, CHX où X est un groupe qui s'éloigne, ou >C=O; tandis que $Y^1$ et $Y^2$ représentent chacun indépendamment l'un de l'autre un atome d'hydrogène ou un groupe protecteur du groupe hydroxyle.

10. Carbohydrate selon la revendication 9, dans lequel Q représente CHOH.

11. Carbohydrate selon la revendication 9 ou 10, ce carbohydrate ayant la même configuration que le ribose.

12. Carbohydrate selon la revendication 9, dans lequel Q représente >C=O.

13. Carbohydrate selon la revendication 9, dans lequel X de CHX est un groupe sulfonate qui s'éloigne, un atome de chlore ou un atome de brome.

14. Carbohydrate selon l'une quelconque des revendications 9 à 13, dans lequel $Y^1$ et $Y^2$ représentent chacun un atome d'hydrogène, ou un dérivé protégé de ce carbohydrate.

15. Le 2-désoxy-2,2-difluoro-1-oxo-ribose ou un de ses dérivés protégés.

16. le 1-méthane-sulfonyloxy-2-déoxy-2,2-difluororibose ou un de ses dérivés protégés.

17. Le 2-désoxy-2,2-difluororibose ou un de ses dérivés protégés.

18. Procédé de préparation d'un composé de formule (I) comme défini dans les revendications 1 à 8, caractérisé en ce qu'il consiste à éliminer les groupes protecteurs d'un nucléoside de formule (I) protégé de manière correspondante.

19. Procédé de préparation d'un composé de formule (I) comme défini dans les revendications 1 à 8, caractérisé en ce qu'il consiste à coupler un composé de formule (II), comme défini dans la revendication 9, dans laquelle Q représente CHX, tandis que $Y^1$ et $Y^2$ représentent chacun un groupe hydroxyle, ou un dérivé protégé de ce composé de formule (II), avec une base comme défini dans la revendication 1, et éventuellement éliminer les groupes protecteurs.

20. Procédé de préparation d'un composé de formule (II) comme défini dans la revendication 9, dans laquelle Q représente CHOH, ou d'un dérivé protégé de ce composé, caractérisé en ce qu'il consiste à réduire un composé de formule (II) dans laquelle Q représente $>C=O$, ou un dérivé protégé de ce composé.

21. Nucléoside de formule (I) ou un de ses sels pharmaceutiquement acceptables selon l'une quelconque des revendications 1 à 8, en vue de l'utiliser comme agent antiviral.

22. Nucléoside de formule (I) ou un de ses sels pharmaceutiquement acceptables selon l'une quelconque des revendications 1 à 8, en vue de l'utiliser pour le traitement d'infections herpès.

23. Formulation pharmaceutique comprenant, come ingrédient actif, un nucléoside de formule (I), ou un de ses sels pharmaceutiquement acceptables, selon l'une quelconque des revendications 1 à 8, en association avec un ou plusieurs supports ou véhicules physiologiquement acceptables pour ce nucléoside ou ce sel.

**Revendications pour l'Etat contractant: AT**

1. Procédé de préparation d'un nucléoside de formule (I):

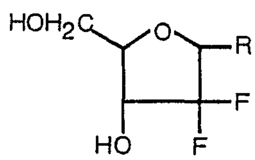

(I),

d'un de ses dérivés protégés ou d'un de ses sels pharmaceutiquement acceptables, formule dans laquelle R est une base d'une des formules suivantes:

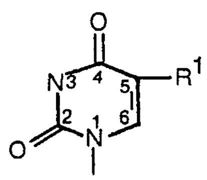

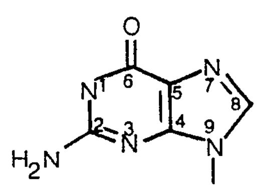

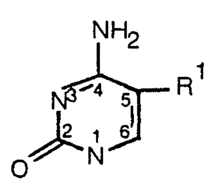

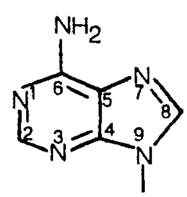

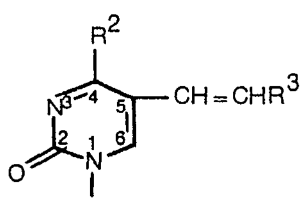

où

$R^1$ représente un atome d'hydrogène, un groupe méthyle, un atome de brome, un atome de fluor, un atome de chlore ou un atome d'iode;

$R^2$ représente un groupe hydroxyle ou un groupe amino;

$R^3$ représente un atome de brome, un atome de chlore, ou un atome d'iode,

caractérisé en ce qu'il consiste à éliminer les groupes protecteurs d'un nucléoside de formule (I) protégé de manière correspondante.

2. Procédé de préparation d'un composé de formule (I) ou d'un de ses sels pharmaceutiquement acceptables, comme défini dans la revendication 1, caractérisé en ce qu'il consiste à coupler un composé de formule (II):

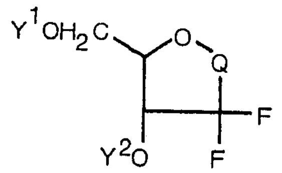

( II )

dans laquelle Q représente CHX où X est un groupe qui s'éloigne, tandis que $Y^1$ et $Y^2$ représentent chacun indépendamment l'un de l'autre un atome d'hydrogène ou un groupe protecteur du groupe hydroxyle, avec une base comme défini dans la revendication 1, et, si on le désire, éliminer tout groupe protecteur éventuel.

3. Procédé de préparation d'un nucléoside selon la revendication 1 ou 2, caractérisé en ce que la fraction carbohydrate est le groupe 2-désoxy-2,2-difluororibosyle.

4. Procédé de préparation d'un nucléoside selon l'une quelconque des revendications 1 à 3 ou d'un de ses sels pharmaceutiquement acceptables, caractérisé en ce que R représente

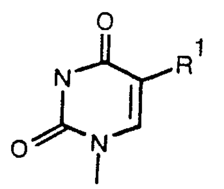

ou

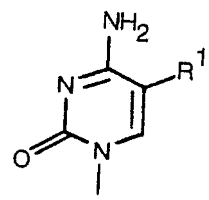

où $R^1$ a la signification définie dans la revendication 1.

5. Procédé de préparation d'un nucléoside selon l'une quelconque des revendications 1 à 4 ou d'un de ses sels pharmaceutiquement acceptables, dans lequel R représente

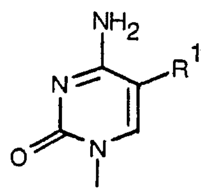

et $R^1$ a la signification définie dans la revendication 1.

6. Procédé selon l'une quelconque des revendications 1 à 4 en vue de préparer le 1-(5-méthyl-2,4-dioxo-1H,3H-pyrimidin-1-yl)-1,2-désoxy-2,2-difluororibose ou un de ses sels pharmaceutiquement acceptables.

7. Procédé selon l'une quelconque des revendications 1 à 5, en vue de préparer le 1-(4-amino-2-oxo-1H-pyrimidin-1-yl)-2-désoxy-2,2-difluororibose ou un de ses sels pharmaceutiquement acceptables.

8. Procédé selon l'une quelconque des revendications 1 à 5 en vue de préparer le 1-(4-amino-5-iodo-2-oxo-1H-pyrimidin-1-yl)-2-désoxy-2,2-difluororibose ou un de ses sels pharmaceutiquement acceptables.

9. Procédé selon l'une quelconque des revendications 1 à 4 en vue de préparer le 1-(2,4-dioxo-5-fluoro-1H,3H-pyrimidin-1-yl)-2-désoxy-2,2-difluororibose ou un de ses sels pharmaceutiquement acceptables.

10. Procédé de préparation d'un composé de formule (II):

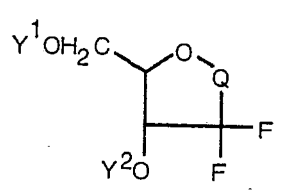

( II )

dans laquelle Q représente CHOH, tandis que $Y^1$ et $Y^2$ représente chacun indépendamment l'un de l'autre un atome d'hydrogène ou un groupe protecteur du groupe hydroxyle, caractérisé en ce qu'il consiste à réduire un composé de formule (II) dans laquelle Q représente >C=O, ou un dérivé protégé de ce composé de formule (II) et, si on le désire, éliminer tout groupe protecteur présent.

11. Procédé de préparation d'un composé de formule (II) dans laquelle Q représente CHOH, selon la revendication 10, caractérisé en ce que le produit a la même configuration que celle du ribose.

12. Procédé de préparation du 2-désoxy-2,2-difluororibose ou d'un de ses dérivés protégés selon la revendication 10 ou 11.

13. Nucléoside de formule (I) ou un de ses sels pharmaceutiquement acceptables, préparé par un procédé selon l'une quelconque des revendications 1 à 9.

14. Carbohydrate de formule (II) ou un de ses dérivés protégés, préparé par un procédé selon l'une quelconque des revendications 10 à 12.